# EUROPEAN PATENT APPLICATION

(11) **EP 2 837 932 A1**
(43) Date of publication of application: **18.02.2015**
(21) Application number: 13004078.5
(22) Date of filing: 16.08.2013
(51) Int. Cl.: G01N 27/12

(54) **Annealing process for integrated metal oxide gas sensors**

(71) Applicant: Sensirion AG, 8712 Stäfa (CH)
(72) Inventor: Mücke, Ulrich, 8037 Zürich (CH); Bürgi, Lukas, 8049 Zürich (CH); Mayer, Felix, 8712 Stäfa (CH)
(74) Representative: Mirza, Akram Karim

(57) **Abstract**

A method of manufacturing an integrated metal oxide gas sensor is described including the steps of depositing a composition with metal oxide particles or precursors thereof at the desired location on a substrate including electronic components followed by a step of heating the substrate whereby the heating step is designed such that it creates a local temperature difference between the location of the deposited composition and the location of more temperature sensitive parts of the sensor such as the electronic components and is interrupted before the temperature of the substrate at the location of the electronic components reaches a threshold above which the more sensitive parts can be damaged.

## Description

### FIELD OF THE INVENTION

The present invention relates to a method of manufacturing an integrated chemical sensor, particularly a gas sensor using metal oxide(s). The sensor is located on a substrate with electronic components embedded therein.

### BACKGROUND OF THE INVENTION

Metal oxide (MOX) gas sensors are devices based on thermally assisted chemical reactions between the metal oxide sensing layer and the gas to be detected. Typically, MOX sensors are operated at a temperature ranging from 150°C to 450°C to allow for the oxidation of gas in contact with the layer. Often the heat for achieving the operating temperature is provided by integrated heaters.

In an integrated MOX gas sensor, particularly a CMOS-type integrated gas sensor, the sensing layer is placed onto the surface of a silicon based substrate. The substrate will usually include electronic circuitry for the operation of the sensor and data acquisition and processing of the sensor data. In such sensors the heaters are integrated into or placed onto the substrate near the MOX layer, typically with resistive heating elements made of Pt or poly-Si or tungsten. The silicon in the vicinity of the sensor is usually microstructured or micromachined in a MEMS-type processing to create a thin membrane around the MOX layer, electrodes and the heating elements together with contacts to allow for a signal or power transfer between the sensor and the rest of the electronic circuit.

The membrane is thinned to provide a low heat capacity and thermal insulation so that the heater can heat the sensing layer without affecting the electronic circuit elements, which typically have a temperature envelope for safely operating below the operating temperatures of the sensor.

An efficient method of manufacturing gas sensors is seen in preparing wafers with a large number of identical sensors. It is known for example from the United States patents no. 5821402 and the commonly owned no. 7955645B2 to steer vapor deposition to localized zones of increased temperature as location for a preferred deposition of metal oxide material.

In a different method the MOX material is delivered to the substrate at the desired location using drop delivery or ink jetting tools. While these methods already provide a deposition process which confines the MOX material to the desired locations on the substrate, the delivery process is performed using a suspension of which the MOX material is only one component among others such as solvents, surfactants and the like. To rid the deposited material from these additional components and to create a homogenous MOX layer it is known to use a thermal treatment. This heat treatment can be regarded as an evaporation and/or a burn-off of organic matter or solvent sometimes followed by an annealing step to improve particle-to-particle connectivity, applied to the drop after deposition to remove residuals from the deposition process and to chemo-mechanically stabilize the remaining MOX particles. The heat treatment process can require temperatures up to 600°C.

It is therefore an object of the invention to provide a thermal treatmentprocess as part of a manufacturing process of integrated metal oxide gas sensors.

### SUMMARY OF THE INVENTION

Hence, according to a first aspect of the invention, there is provided a method of manufacturing an integrated metal oxide gas sensor including the steps of depositing a composition including metal oxide particles at the desired location on a substrate including electronic components followed by a step of heating the substrate to remove unwanted residues from the composition and/or provide a stabilization of the remaining layer of metal oxide on the substrate. The heating step is designed such that it creates a local temperature difference between the location of the deposited composition and the location of the electronic components such that the temperature of the substrate at the location of the electronic components remains below a threshold above which the electronic components can be damaged, while the area of deposition can be heated to temperatures above such a threshold.

The composition including metal oxide particles can be suspensions of metal oxide particles, solutions of salts of metal, sol-gel compounds and other suitable pre-cursor mixtures designed to form metal oxide layers after a heat treatment.

The temperature difference can be a difference in temperature between two lateral locations on the substrate, or between two vertical locations or layers of the substrate or both.

The method is preferably applied such that the deposition of the composition of MOX material is performed at a temperature below 100°C, preferably below 50°C. The temperature difference can have a limiting temperature at its upper end of 250°C to 800°C, preferably of 300°C to 500°C.

Either the deposition or the heating step can be performed in an ambient, clean either dry or humid (with a relative humidity of between 10 and 95 per cent) air environment or in an inert gas, oxygen-enriched or pure oxygen atmosphere. The latter can be important to allow the burn-off of carbon-based residues at lower temperatures than under ambient air. In some cases the air may include other reactive (oxidizing or reducing) gas components such as methane.

The heating process is preferably applied to heat the substrate including the composition deposited on it from an initial temperature within a range from ambient temperature to about 150°C. Thus, the substrate can be heated prior to the inhomogeneous heating as described above to a homogeneous temperature within a range from ambient temperature to about 150°C, e.g., 80°C. This homogenous pre-heating step step can be useful to evaporate at least some of the organic components in the composition used in the deposition process.

The heating process can include a process using the differences in heat capacities and/or heat coupling to the surrounding, e.g., to a handling table (chuck), between the area with the deposited MOX material and the location of the electronic circuitry. The heating process can include the use of a local heat source such as an infrared or ultraviolet laser or laser diode. The heating process can include the use of global heat source applied using a structured mask. A cooling facility can be used to limit the temperature of areas of the substrate outside the location of the deposited composition.

The heat source can be operated in a continuous mode or in a pulsed or flashed mode. In a preferred embodiment of the invention, the heat source is tuned or filtered to emit radiation in a range of wavelengths where either the metal oxide, the composition, the upper cover layers of the substrate, i.e., SiOx or SiNx, or the electrodes exhibit a strong absorption.

A local heat source is thereby understood as a heat source which initially delivers heat only to a confined spot before spreading within the substrate through normal thermal conduction processes. A global heat source is understood to heat the whole or most of the substrate including the location of the deposited composition and the location of the electronic components.

A preferred local heating process makes use of heater elements integrated into the sensor for the ultimate purpose of bringing the MOX layer to its operating temperature during a measurement.

The heating process can be applied at wafer level or after a dicing step used to cutting the wafer into individual sensors or small groups of sensors.

The deposition process preceding the heating step includes preferable a contactless deposition such as ink jet printing using a composition of metal oxide(s) as an ink. The deposition and the heating process are preferably performed at different locations, for example at different locations along an assembly or wafer handling line.

The MOX material can be tin oxide, tungsten oxide, gallium oxide, indium oxide, zinc oxide, which preferably may be applied in a high temperature environment. The layer may be further doped with heteroatoms.

The electronic components can be passive or active electronic elements. Preferably they include CMOS circuitry for control and data acquisition and processing.

The above and other aspects of the present invention together with further advantageous embodiments and applications of the invention are described in further details in the following description and figures.

### BRIEF DESCRIPTION OF THE FIGURES

FIGs. 1A and 1B are a schematic perspective view and a cross-section, respectively, of a metal oxide gas sensor;
FIG. 2 illustrates manufacturing steps of a metal oxide gas sensor in accordance with an example of the invention;
FIGs. 3A - 3C illustrate details and variants of a manufacturing step in FIG. 2; and
FIGs. 4A - 4B illustrate further variants in accordance with an example of the invention.

### DETAILED DESCRIPTION

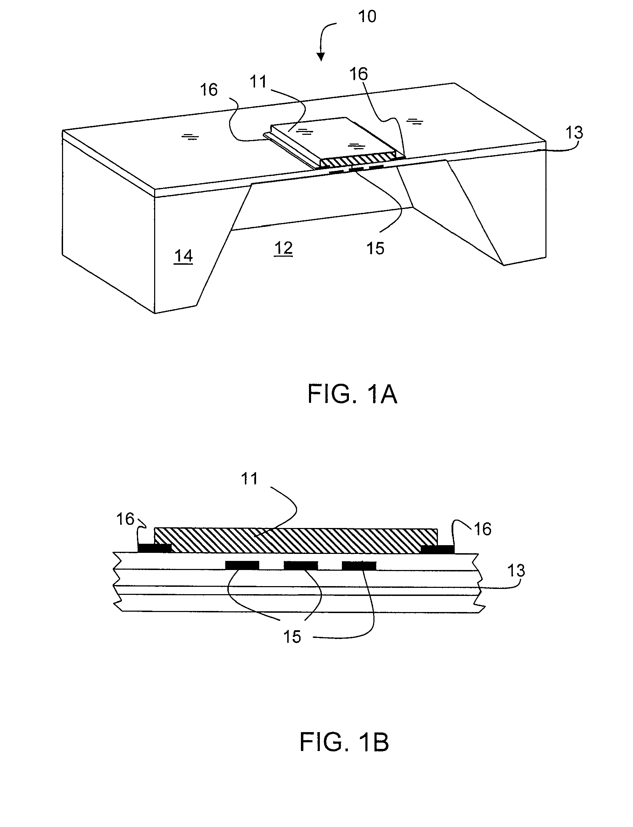

A known gas sensor 10 with a sensing layer 11 of metal oxide is shown in FIGs. 1A and 1B. The sensor is integrated with a CMOS circuitry (not shown) on a single chip. Parts of the CMOS layers 13 and handle layer 14 required for the CMOS circuit are etched away in a microstructuring or micromachining (MEMS) process to form a cavity 12 at the location of the sensor. The remaining layers 13 form a thin membrane to support the actual sensor 10.

The material of the electrodes is typically a metal, for example Pt, Au, Al or W. The metal-oxide used can be tin oxide, tungsten oxide, gallium oxide, indium oxide, or zinc oxide. As described in further detail, a micro electro-mechanical system or heat source can also be integrated within or below the sensor. The sensor is built with its own CMOS circuitry for control and readout. The physical dimensions of the substrate including the CMOS circuit and the MEMS sensor are less than 5mm x 5mm.

Embedded within the layers 13 are conducting elements forming a heater 15 to provide a local source of heat to heat the metal oxide 11 during operation of the sensor 10. The membrane structure 12 provides an inherent thermal insulation for the rest of the substrate with the CMOS circuit. The metal oxide layer 11 is contacted by two conductive electrodes 16 and hence acts as a resistor. In the presence of an analyte this resistance changes thereby providing a measure of the concentration of the analyte in the immediate vicinity of the metal oxide layer.

Methods of manufacturing the above or similar metal oxide sensors are described in the following figures using the same numerals for elements common with or similar to those appearing already in FIGs. 1A and 1B.

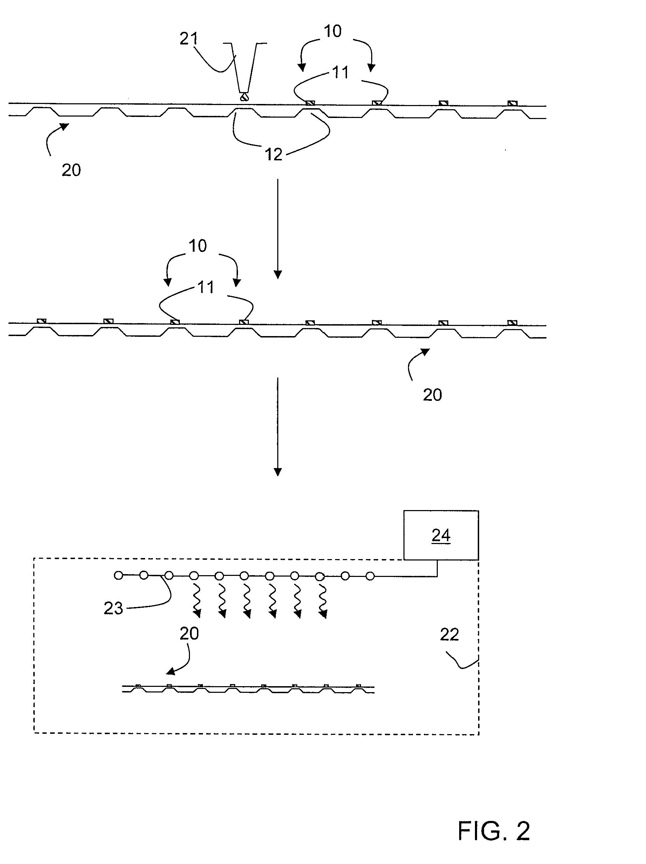

In FIG. 2 there is shown a CMOS-type wafer 20 with embedded CMOS electronic circuits. The wafer has been etched in a MEMS or microstructuring step to form the cavities 12 to create membranes onto which the MOX layer 11 is mounted. In the top section of the figure, the wafer 20 is shown with a schematic cross-section through several sensor elements 10.

A nozzle 21 representing a drop deposition system is used to deposit a layer of MOX material 11 onto the membrane areas 12 of the wafer. The size of the drop and the position of the nozzle or capillary 21 relative to the wafer 20 are selected so as to ensure the appropriate placing of the resulting MOX layer 11 at its designated position. The step can be performed typically under ambient or clean room conditions only, and does not require a reactive gas environment as for example chemical vapor deposition methods. Moreover, it is not necessary to heat the wafer 20 or parts of the wafer during this deposition step. The wafer is maintained during the deposition at its normal handling temperature, e.g. temperatures below 100°C, even below 50°C or at ambient temperatures.

The middle section of FIG. 2 shows a wafer 20 after the deposition process but prior to the thermal treatment process. The MOX layers 11 are placed on the designated areas on the wafer 20. The wafer 20 is maintained during the transfer from the deposition stage to the heat treatment stage at its normal handling temperature, e.g. temperatures below 100°C, even below 50°C or at ambient temperatures.

It can be advantageous to precede the inhomogeneous heat treatment stage with a homogeneous heating stage during which the substrate is homogeneously heated to a temperature above the deposition temperature but below the temperature limits for the electronic components. At this temperature, e.g. at 80°C, at least some of those components can be removed without providing cold spots on the substrate at which they can re-condensate.

For heat treatment the wafer is transferred into a heating zone or an oven area 22 as shown in the bottom section of FIG. 2. Typically the heating zone is at a different location along the process line than the deposition location, where the above deposition steps are performed The oven includes a heat source 23 with a control 24. The heat source can be a global heat source, such as a conduction, convection heater (with or without fan) or radiation heater, such as electrical hot plates, quartz tubes or halogen radiators, designed to heat the complete sensor 10 or wafer 20. Wide IF radiators as commercially available (e.g., Elstein) are for example well suited for heating the full wafer. Alternatively the heat source can be a local heat source focusing radiation on the area of the MOX layer. However, irrespective of the type of heat source and its control and other elements to be described below, the oven stage is designed to generate a temperature gradient in the area of the sensor between the location of the MOX layer and the CMOS electronic components.

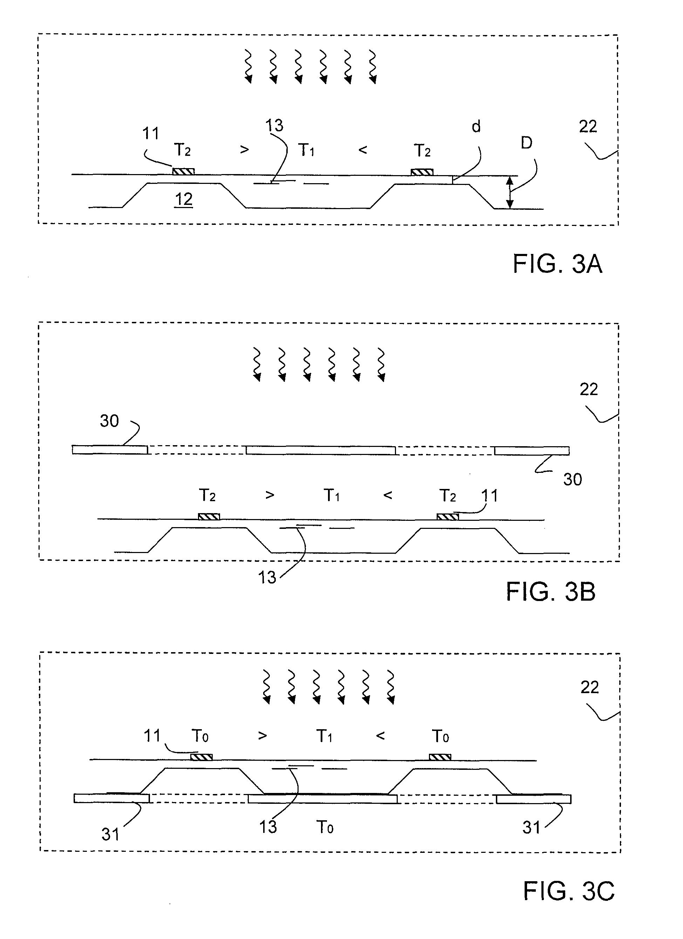

Various examples of generating such a temperature gradient in the area of the sensor between the location of the MOX layer 11 and the CMOS electronic components 13 using a global heat source are illustrated in FIGs. 3A to 3C. In the figures the temperature difference is denoted by a temperature T₁ of the electronic parts 13 and a higher temperature T₂ of the MOX layer 11. The temperature T₂ for the heat treatment, chemo-mechanical stabilization or annealing of the MOX material is in the range of 250°C to 600°C, or 300°C to 500°C, and typically about 100°C to 150°C higher than the later operating temperature of the MOX sensor. The temperature T₁ depends on the temperature sensitivity of the CMOS components integrated into the substrate. It is typically in the range of 100°C to 200°C for an extended period of time and up to typically 400°C for a few minutes.

Other limits are however applicable in cases where the manufacturing processes are altered. If for example the sensors are manufactured using a lead-frame with mold packaging where the CMOS dies are wirebonded onto a lead frame and surrounded by a polymer with openings and the sensor elements are then printed onto the dies in the molded lead frames and temperature treated with a temperature differential as above the maximum temperatures T₁ are 150°C for periods of about 1000h, 200°C für a period not exceeding ten hours or 250°C für several minutes or even seconds. In cases where a glue is applied to combine two or more wafer or dies in the manufacturing of the sensor, the temperature stability of the glue needs to be considered.

Referring now to the example of FIG. 3A, the heat source is assumed to be a global heat source radiating with the same power onto all parts of the wafer. With such a heat source, the differences in heat capacities can be used such that the membrane area above the cavity 12 is heated to T₂ while the remaining parts of the substrate are heated to the lower temperature T₁. Thus even when the substrate is exposed to a global, homogeneous heater which transfers an equal amount of heat per unit area to the substrate it is possible to create and maintain a significant difference in temperature for some time.

Depending on the design of the heating process the temperatures or temperature differences can be maintained through a constant or a pulsed heat source 23.

The heat source can be tuned to emit radiation with at least a local maximum at around 10 microns. This is a range where metal oxide(s), the composition, the upper cover layers of the substrate, i.e., SiOx or SiNx, absorb strongly. When operated in a pulsed or flashed mode, using for example pulse width of 1 to 10000 microsecond and a pulse energy in the range of 1 - 500 J/cm2 the process can be controlled such that only the deposited layer or only the top layer of the substrate are heated to higher temperatures while the lower layers substrate including the CMOS circuit elements remains at a lower temperature. Thus the heating maintains a vertical temperature difference on the substrate. Suitable lamps operating in the IR, near-IR or visible spectrum for such a heating process are commercially available from companies such as Novacentrix, Xenon Corp., or DFT Technology. To achieve a desired emission spectrum, the heat source can be combined with a filter. It is also feasible to tailor the radiation to the absorption of the electrode material, which has maxima typically in the visible to near-IR spectrum.

In the example of FIG. 3B, a mask 30 is placed between the heat source 23 and the wafer 20. The mask lets the radiation pass in the areas above the membrane while acting as a heat sink above the wafer body 13. The mask is typically made of a mechanically stable material with good heat conduction properties and connected to a cooling device (not shown) to remove the absorbed heat or cooled by natural convection.

In the example of FIG. 3C, a cooling body 31 is brought in contact with the back of the wafer 20. The temperature difference can be generated exploiting the different heat couplings between the substrate and a support structure, such as a metal table or wafer chuck, at the regions of original thickness, and the regions under the membrane. The latter are separated from the support structure by an air gap. The air gap provides a significantly increased heat insulation than the silicon wafer material, which is a good heat conductor.

The cooling body is cooled to a temperature Tₒ below T₁ and maintains the temperature T₁ in the parts of the wafer with electronic components 13. The membrane with the MOX layer 11 is heated by the heat source to the temperature T₂. The cooling body can be for example a flat unstructured metal sheet or structured with openings at the membrane areas and it can be in turn connected to a cooling device.

With the use of a cooling body the temperature difference can be stabilized for much longer periods than without a cooling body. In the latter case the temperature difference may be maintained only for several seconds compared to practically hours when using a cooling body.

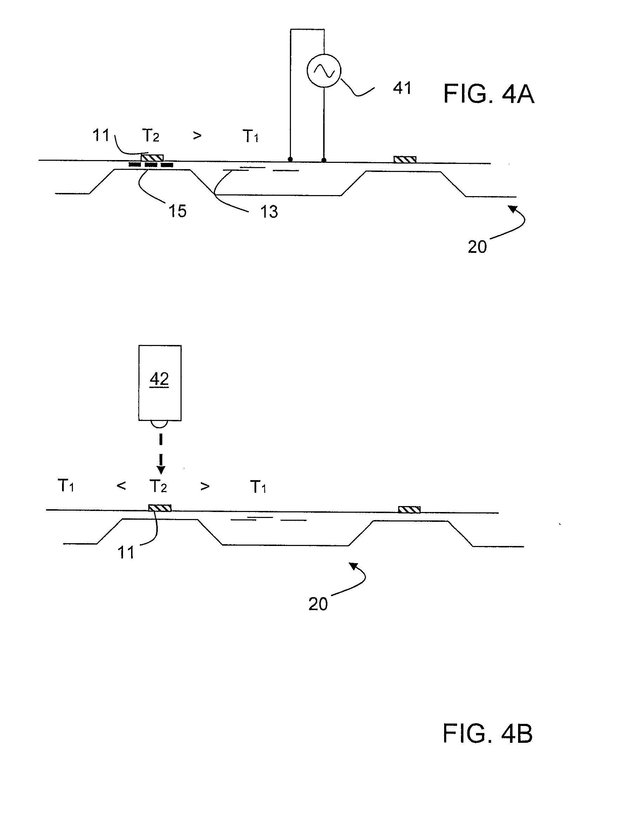

The use of local heat sources for the purpose of heat-treating a layer of MOX material 11 as deposited in a previous manufacturing step are illustrated in the examples of FIGs. 4A and 4B.

In FIG. 4A the heating elements 15 which are embedded in or mounted onto the substrate are used to heat the MOX layer 11 to the temperature T₂. To supply the heating elements 15 with electric power a power source 41 is provided to access contact points on the substrate. The heating process is similar to the use of the heaters during the later normal operation as gas sensor, however, controlled such that the MOX layer is heated to a temperature T₂ higher than the normal operating temperature of the sensor The process can be performed in parallel on several or all sensors of the wafer 20 simultaneously.

In the example of FIG. 4B an IR laser light is used to confine the initial heat transfer to the vicinity of the MOX layer 11. The heat pulse intensity and duration is measured such that MOX layer 11 is heated to the desired temperature T₂. The laser 42 can be a diode laser or a CO₂ laser. The laser light can be focused onto the MOX layer using glass fibers or optical elements or mirrors. By splitting the laser light or by using several lasers the process can be performed in parallel on several or all sensors of the wafer 20 simultaneously.

Some or all of the above examples can be used in combination. It is for example possible to use a global heat source to heat the wafer to a common temperature below or near T₁ and use a local heat source to heat the area around the MOX layers to the desired temperature T₂. Each of the processes can use masks or coolers as required.

The steps illustrated in FIG. 2 are shown as full wafer handling steps. However it is also possible to dice the wafer into individual sensors or groups of sensors. Depending on the overall process efficiency the dicing of the wafer can take place prior to the deposition and annealing steps or after the deposition step but before the heating step. The diced parts are typically handled in an assembly line process of which the heating is one of several stages. Rather than using a batch process the oven 22 can have an entry and an exit to allow for a continuous processing/heating of many sensors.

While there are shown and described presently preferred embodiments of the invention, it is to be understood that the invention is not limited thereto but may be otherwise variously embodied and practised within the scope of the following claims.

## Claims

1. A method of manufacturing an integrated metal oxide gas sensor comprising the steps of
- depositing a composition including metal oxide particles (MOX) or pre-cursors of metal oxides at the desired location on a substrate including electronic components;
- followed by a step of heating the substrate, wherein the heating step generates a local temperature difference between the location of the deposited composition and other locations on the substrate such that the temperature of the substrate at the other locations remains below a temperature threshold above which damages to the other locations occur.

2. The method of claim 1, wherein the deposition of the composition is performed at a temperature below 100°C.

3. The method of claim 1 or 2, wherein the temperature difference has a limiting temperature at its upper end selected from a range of 250°C to 600°C.

4. The method of any of the preceding claims, wherein the deposition and/or the heating is performed in an ambient, clean , dry or humid air environment or in an inert or oxygen-enriched or reducing gas atmosphere.

5. The method of any of the preceding claims, wherein the heating process is applied to heat the substrate including the composition deposited on it from an initial temperature within a range from ambient temperature to about 150°C.

6. The method of any of the preceding claims, wherein the heating process uses a heat source in a continuous, or pulsed or flashed mode.

7. The method of any of the preceding claims, wherein the heating process uses a heat source with an emission spectrum tuned to the absorption of parts of the upper layers of the substrate.

8. The method of any of the preceding claims, wherein the heating process uses a heat source transferring heat essentially homogeneous to the substrate.

9. The method of claim 8, wherein the heating process is controlled such that the temperature gradient is generated using a difference in the heat coupling between the substrate and the surrounding at the location of the deposited composition and the other locations of the substrate.

10. The method of claim 8, wherein the temperature difference is generated using a heat shielding between the heat source and the substrate at the other locations of the substrate.

11. The method of any of the preceding claims, wherein the heating process uses a heat source transferring heat preferably to the location of the deposited composition.

12. The method of claim 11, wherein the heating process includes the use of heating elements integrated within the substrate.

13. The method of claim 11, wherein the heating process uses beams of radiation.

14. The method of claim 11, wherein the heating process uses beams of radiation of a beam radius of 300 microns or less.

15. The method of any of the preceding claims, wherein the heating process uses a heat source transferring heat essentially homogeneous to the substrate and a heat source transferring heat preferably to the location of the deposited composition.

16. The method of any of the preceding claims, wherein the depositing step includes a step of ejecting droplets of the composition from a nozzle across a gap onto the substrate.

17. The method of any of the preceding claims, wherein the other locations are locations of integrated electronic components within the substrate, particularly CMOS components.
